(19)

Europäisches
Patentamt
European
Patent Office
Office européen
des brevets

(11) **EP 2 910 925 A1**

(12) **EUROPEAN PATENT APPLICATION**

published in accordance with Art. 153(4) EPC

(43) Date of publication:
**26.08.2015 Bulletin 2015/35**

(51) Int Cl.:
***G01N 21/17*** *(2006.01)*

(21) Application number: **13846414.4**

(86) International application number:
**PCT/JP2013/077299**

(22) Date of filing: **08.10.2013**

(87) International publication number:
**WO 2014/061498 (24.04.2014 Gazette 2014/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **17.10.2012 JP 2012229996**

(71) Applicant: **Sumitomo Electric Industries, Ltd.
Osaka 541-0041 (JP)**

(72) Inventors:
• **TANAKA, Masato
Yokohama-shi, Kanagawa 244-8588 (JP)**
• **SOGAWA, Ichiro
Yokohama-shi, Kanagawa 244-8588 (JP)**

(74) Representative: **Hoffmann Eitle
Patent- und Rechtsanwälte PartmbB
Arabellastraße 30
81925 München (DE)**

(54) **OPTICAL TOMOGRAPHIC IMAGE ACQUIRING DEVICE**

(57)      An optical tomographic image acquiring device is provided which can suppress the occurrence of an artifact, and which can obtain an exact optical tomographic image of a measurement object. The optical tomographic image acquiring device 1 includes a light source 11, a detector 12, an analysis unit 13, a circulator 20, a coupler 30, condensing lenses 41 and 42, optical fibers 51 and 52, and a reference mirror 91. Given that $\delta k$ is a maximum value of intervals in wavenumber of lights received by adjacent two light receiving elements in the detector 12, an optical path length $L_{0ref}$ from the coupler 30 to the detector 13 through a path going to and returned from the reference mirror 91 and an optical path length $L_{0obj}$ from the coupler 30 to the detector 13 through a path going to and returned from the measurement object 92 satisfy $|L_{0obj} - L_{0ref}| < \pi/\delta k$, and an optical path length $L_{1ref}$ from the coupler 30 to the detector 13 through a path going to and returned from the condensing lens 41 and an optical path length $L_{1obj}$ from the coupler 30 to the detector 13 through a path going to and returned from the condensing lens 42 satisfy $|L_{1obj} - L_{1ref}| > \pi/\delta k$.

FIG. 2

**Description**

Technical Field

The present invention relates to an optical tomographic image acquiring device.

Background Art

The optical tomographic image acquisition technology on the basis of Optical Coherence Tomography (OCT) is able to measure a reflection intensity distribution in the direction of depth of a measurement object by utilizing optical interference. The optical tomographic image acquisition technology has recently been applied to biological measurement because of its capability of imaging an internal structure of the measurement object in a non-invasive manner with a high spatial resolution.

In an optical tomographic image acquiring device on the basis of OCT, light output from a light source is branched into two beams of reference light and measurement light. Reflected light generated from a reference mirror upon the reference mirror being irradiated with the reference light and diffusely-reflected light generated from a measurement object upon the measurement object being irradiated with the measurement light are caused to interfere with each other. Resulting interference light is detected by a detector. A reflection information distribution (i.e., a one-dimensional optical tomographic image) in the direction of depth of the measurement object is obtained by analyzing the detection result. Furthermore, a two- or three-dimensional optical tomographic image can be obtained by scanning a position of the measurement object where it is irradiated with the light.

An optical tomographic image acquiring device disclosed in US2011/0299091A includes a first coupler, a first circulator, a second circulator, a second coupler, and a detector. The first coupler branches light output from a light source into two beams of reference light and measurement light. The first circulator receives the reference light output from the coupler and outputs the reference light to a reference mirror. The second circulator receives the measurement light output from the coupler and outputs the measurement light to a measurement object. The second coupler combines reflected light generated from the reference mirror and obtained through the first circulator with object light generated from the measurement object and obtained through the second circulator, thus causing the reflected light and the object light to interfere with each other. The detector detects the interference light output from the second coupler. In addition, the disclosed optical tomographic image acquiring device employs an optical fiber in not only a part of a reference optical system, but also in a part of a measurement optical system.

The optical tomographic image acquiring device detects interference light (referred to as "signal interference light" hereinafter) resulting from the interference between the reflected light from the reference mirror and the object light from the measurement object. On that occasion, when light is reflected at the circulators and respective end surfaces of the optical fibers in each of the reference optical system and the measurement optical system, the optical tomographic image acquiring device detects interference light (referred to as "noise interference light" hereinafter) resulting from the interference between those reflected lights as well. In other words, the detector of the optical tomographic image acquiring device detects the signal interference light superimposed with the noise interference light, analyzes the detection result, and obtains an optical tomographic image of the measurement object. In the optical tomographic image obtained in such a case, an artifact attributable to the noise interference light is superimposed as noise on the optical tomographic image of the measurement object.

On the other hand, in an optical tomographic image acquiring device disclosed in JP2012-24551A, aiming to reduce the noise interference light, light incident and emergent surfaces of optical components included in a reference optical system and a measurement optical system are inclined such that reflected lights from the light incident and emergent surfaces of the optical components will not reach the detector. However, the light incident and emergent surfaces cannot be inclined in some of optical components. In that case, the artifact attributable to the reflected lights from those optical components may occur.

SUMMARY OF INVENTION

Technical Problem

An object of the present invention is to provide an optical tomographic image acquiring device, which can suppress the occurrence of an artifact, and which can obtain an exact optical tomographic image of a measurement object.

Solution to Problem

To achieve the above object, the present invention provides an optical tomographic image acquiring device including (1) a light source that outputs light, (2) a branching member that branches the light output from the light source into two beams of reference light and measurement light, (3) a reference optical system including a first optical fiber, a first condensing lens, and a reference mirror, and constituted such that the reference light output from the branching member is guided to propagate through the first optical fiber to be incident on the reference mirror via the first condensing lens, and that reflected light generated from the reference mirror upon the incidence of the reference light is guided to propagate through the first optical fiber via the first condensing lens, (4) a measurement optical system including a second optical fiber and a second condensing lens, and constituted such that the measurement light output from the branching member is guided to propagate through the second optical fiber to be applied to the measurement object for irradiation via the second condensing lens, and that reflected light generated from the measurement object upon the irradiation with the measurement light is guided to propagate as object light through the second optical fiber via the second condensing lens, (5) a detector that receives interference light resulting from interference between the reflected light output from the reference optical system and the object light output from the measurement optical system, and that detects a spectrum of the interference light by a spectrometer including a plurality of light receiving elements set in array, and (6) an analysis unit that obtains an optical tomographic image of the measurement object based on a result detected by the detector.

In the above-described measuring apparatus, let $\delta k$ be a maximum value of intervals in wavenumber of lights received by adjacent two of the plural light receiving elements in the spectrometer, an optical path length $L_{0ref}$ from the branching member to the detector through a path going to and returned from the reference mirror and an optical path length $L_{0obj}$ from the branching member to the detector through a path going to and returned from the measurement object satisfy

$$|L_{0obj} - L_{0ref}| < \pi/\delta k$$

and, an optical path length $L_{1ref}$ from the branching member to the detector through a path going to and returned from the first condensing lens and an optical path length $L_{1obj}$ from the branching member to the detector through a path going to and returned from the second condensing lens satisfy:

$$|L_{1obj} - L_{1ref}| > \pi/\delta k.$$

According to a second aspect, the present invention provides an optical tomographic image acquiring device including (1) a wavelength-variable light source that outputs light, (2) a branching member that branches the light output from the light source into two beams of reference light and measurement light, (3) a reference optical system including a first optical fiber, a first condensing lens, and a reference mirror, and constituted such that the reference light output from the branching member is guided to propagate through the first optical fiber to be incident on the reference mirror via the first condensing lens, and that reflected light generated from the reference mirror upon the incidence of the reference light is guided to propagate through the first optical fiber via the first condensing lens, (4) a measurement optical system including a second optical fiber and a second condensing lens, and constituted such that the measurement light output from the branching member is guided to propagate through the second optical fiber to be applied to the measurement object for irradiation via the second condensing lens, and that reflected light generated from the measurement object upon the irradiation with the measurement light is guided to propagate as object light through the second optical fiber via the second condensing lens, (5) a detector that receives interference light resulting from interference between the reflected light output from the reference optical system and the object light output from the measurement optical system, and that detects intensity of the interference light at each wavelength of the light output from the wavelength-variable light source, and (6) an analysis unit that obtains an optical tomographic image of the measurement object based on a result detected by the detector.

In the above-described measuring apparatus, let $\delta k$ be a maximum value of intervals in wavenumber of light when the intensity of the interference light is detected by the detector, an optical path length $L_{0ref}$ from the branching member to the detector through a path going to and returned from the reference mirror and an optical path length $L_{0obj}$ from the branching member to the detector through a path going to and returned from the measurement object satisfy

$$|L_{0obj} - L_{0ref}| < \pi/\delta k$$

and, an optical path length $L_{1ref}$ from the branching member to the detector through a path going to and returned from the first condensing lens and an optical path length $L_{1obj}$ from the branching member to the detector through a path going to and returned from the second condensing lens satisfy:

$$|L_{1obj} - L_{1ref}| > \pi/\delta k.$$

In the optical tomographic image acquiring device according to the present invention, when the reference optical system includes a first optical component disposed midway the first optical fiber and the measurement optical system includes a second optical component disposed midway the second optical fiber, an optical path length $L_{2ref}$ from the branching member to the detector through a path going to and returned from the first optical component and an optical path length $L_{2obj}$ from the branching member to the detector through a path going to and returned from the second optical component satisfy:

$$|L_{2obj} - L_{2ref}| > \pi/\delta k.$$

In the optical tomographic image acquiring device according to the present invention, when the reference optical system includes a first circulator disposed midway the first optical fiber and branching the reflected light generated from the reflection mirror toward the detector, and a first optical component disposed between the first circulator and the first condensing lens, and the measurement optical system includes a second circulator disposed midway the second optical fiber and branching the object light generated from the measurement object toward the detector, and a second optical component disposed between the second circulator and the second condensing lens, an optical path length $L_{3ref}$ from the branching member to the detector through a path going to and returned from the first optical component and an optical path length $L_{3obj}$ from the branching member to the detector through a path going to and returned from the second optical component satisfy:

$$|L_{3obj} - L_{3ref}| > \pi/\delta k.$$

Advantageous Effects of Invention

According to the present invention, the occurrence of an artifact can be suppressed, and an exact optical tomographic image of the measurement object can be obtained.

BRIEF DESCRIPTION OF DRAWINGS

Figure 1 is a conceptual view of an optical tomographic image acquiring device 1A of a first comparative example.

Figure 2 is a conceptual view of an optical tomographic image acquiring device 1 according to a first embodiment of the present invention.

Figure 3 is a graph plotting the relationship among a measurement range width $z_{max}$, an optical path length difference $\Delta L$, and an optical path length difference $\Delta d$.

Figure 4 is a conceptual view of an optical tomographic image acquiring device 2A of a second comparative example.

Figure 5 is a conceptual view of an optical tomographic image acquiring device 2 according to a second embodiment of the present invention.

Figure 6 is a conceptual view of an optical tomographic image acquiring device 3A of a third comparative example.

Figure 7 is a conceptual view of an optical tomographic image acquiring device 3 according to a third embodiment of the present invention.

Figure 8 is a conceptual view of an optical tomographic image acquiring device 4A of a fourth comparative example.

Figure 9 is a conceptual view of an optical tomographic image acquiring device 4 according to a fourth embodiment of the present invention.

DESCRIPTION OF EMBODIMENTS

Embodiments for carrying out the present invention will be described in detail below with reference to the attached drawings. It is to be noted that the same elements in the drawings are denoted by the identical reference signs and duplicate description of those elements is omitted. The embodiments are described in comparison with corresponding comparative examples.

First comparative example, First embodiment

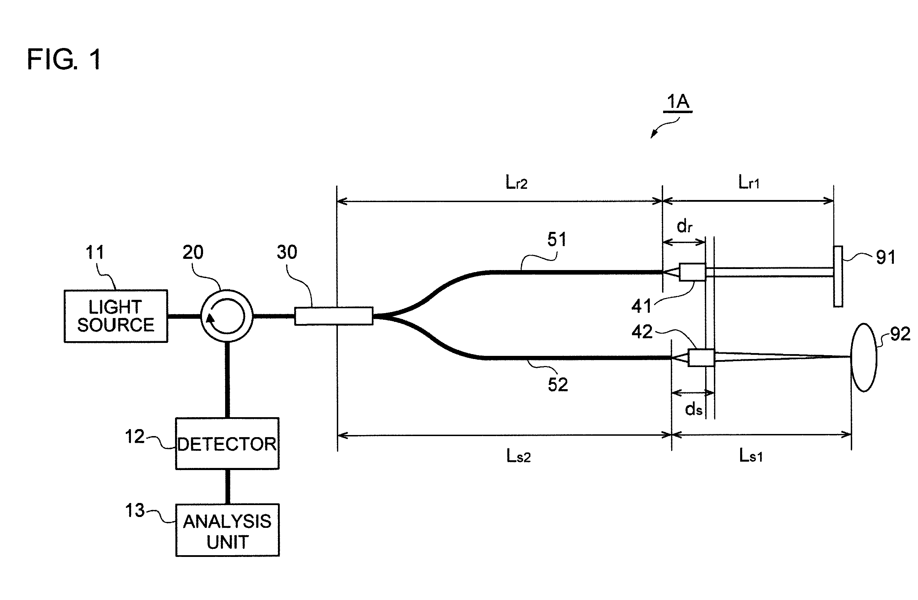

Figure 1 is a conceptual view of an optical tomographic image acquiring device 1A of a first comparative example. The optical tomographic image acquiring device 1A includes a light source 11, a detector 12, an analysis unit 13, a circulator 20, a coupler 30, a first condensing lens 41, a second condensing lens 42, a first optical fiber 51, a second optical fiber 52, and a reference mirror 91. The optical tomographic image acquiring device 1A obtains an optical tomographic image of a measurement object 92 with those components.

The light source 11 outputs light. The circulator 20 receives the light output from the light source 11 and reaching there, and outputs the received light to the coupler 30. The coupler 30 serving as a branching member receives the light output from the light source 11 and reaching there through the circulator 20, and branches the received light into two beams of reference light and measurement light. The coupler 30 outputs the reference light to the first optical fiber 51 and the measurement light to the second optical fiber 52.

A reference optical system includes the first condensing lens 41, the first optical fiber 51, and the reference mirror 91. The optical fiber 51 receives at its one end the reference light output from the coupler 30 and outputs the reference light from the other end after guiding the reference light to propagate therethrough. The condensing lens 41 collimates the reference light output from the optical fiber 51 to be incident on the reference mirror 91. Furthermore, the condensing lens 41 receives reflected light generated from the reference mirror 91 upon the incidence of the reference light, and condenses the reflected light to the end surface of the optical fiber 51. The optical fiber 51 outputs the reflected light to the coupler 30 after guiding the reflected light to propagate therethrough.

A measurement optical system includes the second condensing lens 42 and the second optical fiber 52. The optical fiber 52 receives at its one end the measurement light output from the coupler 30 and outputs the measurement light from the other end after guiding the measurement light to propagate therethrough. The condensing lens 42 condenses the measurement light output from the optical fiber 52 to be applied to the measurement object 92 for irradiation. Furthermore, the condensing lens 42 receives light (object light) reflected from the measurement object 92 upon the irradiation with the measurement light, and condenses the object light to the end surface of the optical fiber 52. The optical fiber 52 outputs the object light to the coupler 30 after guiding the object light to propagate therethrough.

The coupler 30 receives not only the reflected light output from the optical fiber 51 and reaching there, but also the object light output from the optical fiber 52 and reaching there. The coupler 30 outputs interference light, resulting from interference between both the received lights, to the circulator 20. The circulator 20 receives the interference light output from the coupler 30 and reaching there, and outputs the interference light to the detector 12. The detector 12 receives the interference light output from the circulator 20 and reaching there, and detects the interference light. The analysis unit 13 obtains an optical tomographic image of the measurement object 92 based on the result detected by the detector 12.

In Spectrum Domain OCT (SD-OCT), a wide-range light source is used as the light source 11. The detector 12 detects the spectrum of the interference light by a spectrometer including a plurality of light receiving elements set in array.

In Swept-Source OCT (SS-OCT), a wavelength-variable light source is used as the light source 11, and a single light receiving element is used as the detector 12. The detector 12 detects the intensity of the interference light at each wavelength of light output from the wavelength-variable light source 11.

In SD-OCT and SS-OCT, a measurement range in the direction of depth of the measurement object 92 is limited by the Nyquist frequency in discrete Fourier transform that is used in an analysis executed by the analysis unit 13. A measurement range width $z_{max}$ in air is expressed by the following formula (1):

$$z_{max} = \pi/2\delta k$$

$$= \pi N/2\Delta k$$

$$= \pi N/2(2\pi/\lambda_1 - 2\pi/\lambda_2)$$

$$= N\lambda_1\lambda_2/4\Delta\lambda$$

$$\approx N\lambda_0{}^2/4\Delta\lambda. \qquad\qquad ...(1)$$

Here, $\Delta k$ is a band width of the spectrometer or a wave-number variable width of the wavelength variable light source. $\Delta\lambda$ is a band width of the spectrometer or the wavelength variable width of the wavelength variable light source. $\delta k$ is a unit of wave number in the wavelength range of the spectrometer or in the variable range of the wavelength variable light source. $\lambda_1$, $\lambda_2$ and $\lambda_0$ are respectively the shortest wavelength, the longest wavelength, and the center wavelength (= $(\lambda_1 + \lambda_2)/2$) the wavelength range of the spectrometer or in the variable range of the wavelength variable light source. N is the number of spectrum samplings. Assuming $\lambda_0$ = 1310 nm, $\Delta\lambda$ = 90 nm, and N = 1024, for example, the measurement range width $z_{max}$ in air is estimated to be 4.9 mm (= 1024×1265×1355)/4×90) nm).

In the first comparative example, it is assumed that reflected lights are generated from the condensing lenses 41 and 42. Those reflected lights may also reach the detector 12 through the optical fibers 51 and 52, the coupler 30, and the circulator 20.

The distance along an optical path between the coupler 30 and the emergent end of the optical fiber 51 is denoted by Lr2. The distance along an optical path between the coupler 30 and the emergent end of the optical fiber 52 is denoted by Ls2. The distance along an optical path between the emergent end of the optical fiber 51 and the reference mirror 91 is denoted by Lr1. The distance along an optical path between the emergent end of the optical fiber 52 and the measurement object 92 is denoted by Ls1. The distance along an optical path between the emergent end of the optical fiber 51 and an arbitrary reflecting surface associated with the condensing lens 41 is denoted by dr. The distance along an optical path between the emergent end of the optical fiber 52 and an arbitrary reflecting surface associated with the condensing lens 42 is denoted by ds. The effective refractive index of the optical fibers 51 and 52 is denoted by n.

On those assumptions, a difference $\Delta L$ between an optical path length $L_{0ref}$ from the coupler 30 to the detector 12 through a path going to and returned from the reference mirror 91 and an optical path length $L_{0obj}$ from the coupler 30 to the detector 12 through a path going to and returned from the measurement object 92 is expressed by the following formula (2a):

$$\Delta L = |L_{0obj} - L_{0ref}|$$

$$= 2|n(Ls2 - Lr2) + (Ls1 - Lr1)|. \qquad\qquad ...(2a)$$

Furthermore, a difference $\Delta d$ between an optical path length $L_{1ref}$ from the coupler 30 to the detector 12 through a path going to and returned from the reflecting surface associated with the condensing lens 41 and an optical path length $L_{1obj}$ from the coupler 30 to the detector 12 through a path going to and returned from the reflecting surface associated with the condensing lens 42 is expressed by the following formula (2b):

$$\Delta d = |L_{1obj} - L_{1ref}|$$

$$= 2|n(Ls2 - Lr2) + (ds - dr)|. \qquad\qquad ...(2b)$$

In the first comparative example, as expressed by the following formulae (3a),

$$\Delta L/2 < z_{max} \text{ and } \Delta d/2 < z_{max,} \qquad\qquad ...(3a)$$

$\Delta L/2$ and $\Delta d/2$ are both smaller than the measurement range width $z_{max}$. By applying the formula (1), the formula (3a) can be rewritten to the following formulae (3b):

$$\Delta L < \pi/\delta k \text{ and } \Delta d < \pi/\delta k. \qquad \ldots(3b)$$

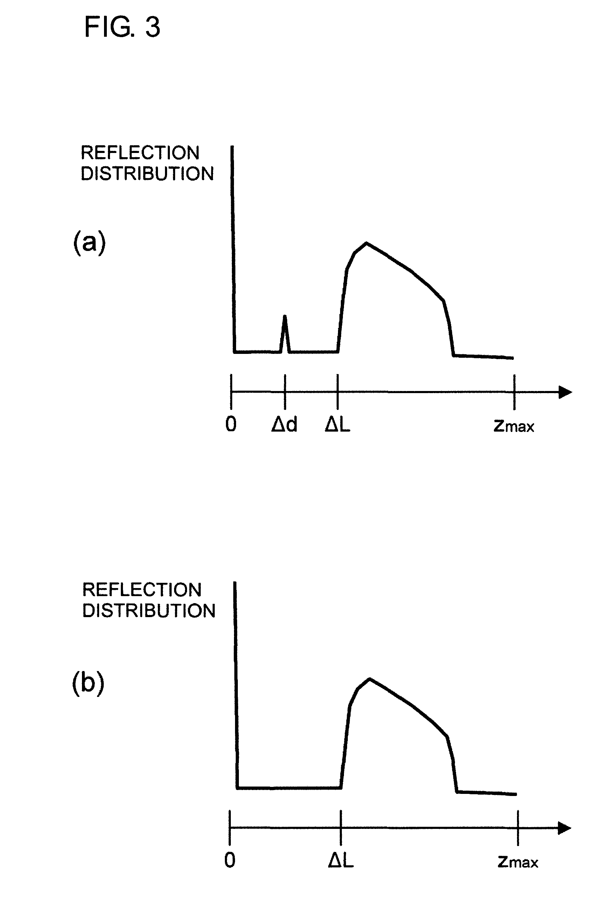

In such a case, an artifact attributable to the reflected lights generated from the condensing lenses 41 and 42 is superimposed as noise on an optical tomographic image of the measurement object 92 (see Fig. 3(a)).

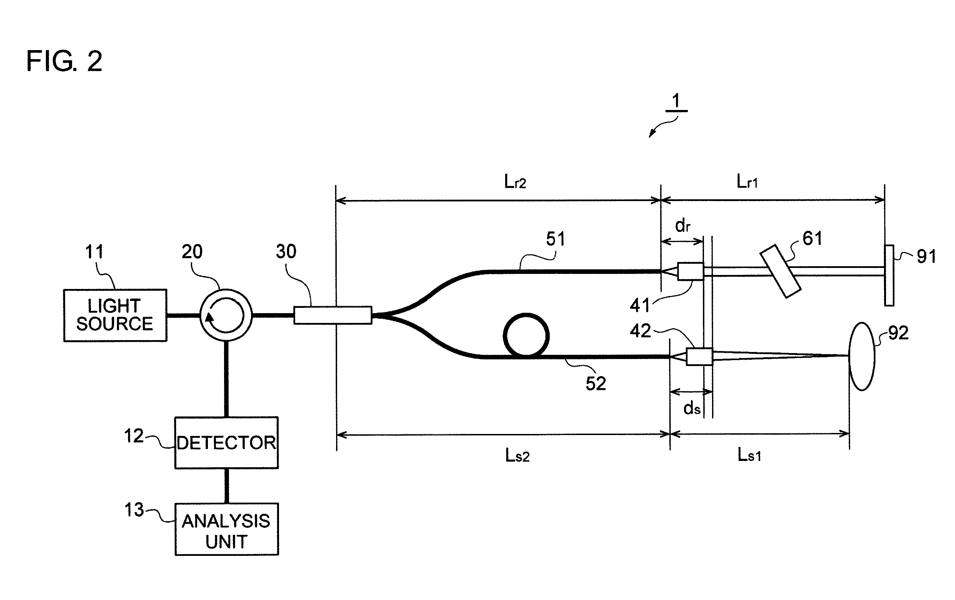

Figure 2 is a conceptual view of an optical tomographic image acquiring device 1 according to a first embodiment. The optical tomographic image acquiring device 1 is different from the optical tomographic image acquiring device 1A of the first comparative example in that the distance Lr1 along the optical path between the emergent end of the optical fiber 51 and the reference mirror 91 in the reference optical system is increased, and that the length Ls2 of the optical fiber 52 in the measurement optical system is also increased. When respective changes of the optical path lengths resulting from increases of Lr1 and Ls2 are equal to other, $\Delta L$ expressed by the above formula (2a) is not changed and the optical tomographic image of the measurement object 91 is obtained in the first embodiment at the same position as that obtained in the first comparative example.

Furthermore, in the first embodiment, as expressed by the following formulae (4a),

$$\Delta L/2 < z_{max} \text{ and } \Delta d/2 > z_{max}, \qquad \ldots(4a)$$

$\Delta L/2$ remains smaller than the measurement range width $z_{max}$, but $\Delta d/2$ is larger than the measurement range width $z_{max}$. By applying the formula (1), the formula (4a) can be rewritten to the following formulae (4b):

$$\Delta L < \pi/\delta k \text{ and } \Delta d > \pi/\delta k. \qquad \ldots(4b)$$

In such a case, the artifact attributable to the reflected lights generated from the condensing lenses 41 and 42 is not superimposed on the optical tomographic image of the measurement object 92 (see Fig. 3(b)).

In the first embodiment, because the optical fiber 51 in the reference optical system and the optical fiber 52 in the measurement optical system have different lengths from each other, the influences of dispersions in the optical fibers 51 and 52 are apt to appear in the optical tomographic image. To cope with that problem, a dispersion compensation element 61 is preferably inserted in the optical path between the condensing lens 41 and the reference mirror 91 in the reference optical system. As an alternative, it is also preferable in SD-OCT or SS-OCT to multiply the interference spectrum by a phase component reversed to that of the dispersion.

Second comparative example, Second embodiment

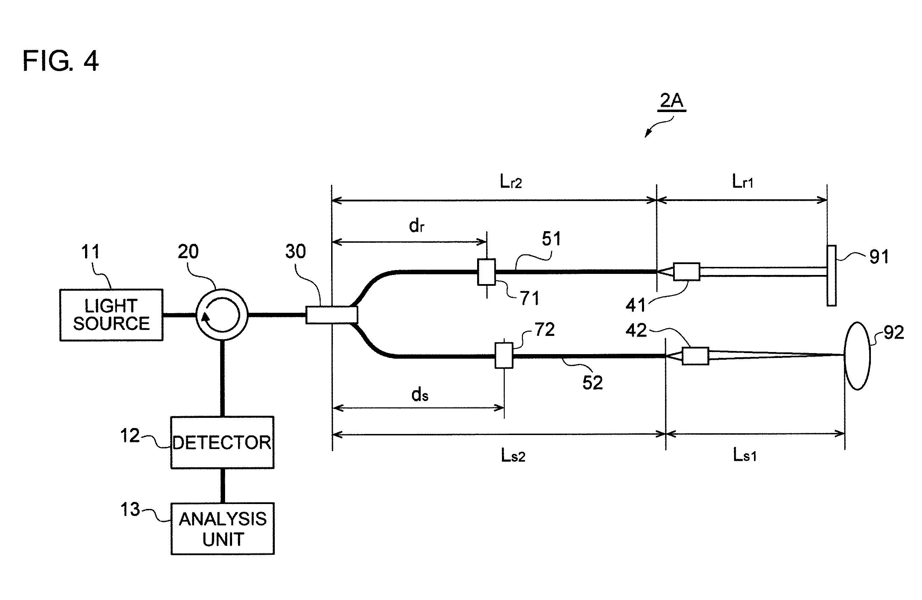

Figure 4 is a conceptual view of an optical tomographic image acquiring device 2A of a second comparative example. The optical tomographic image acquiring device 2A of the second comparative example is different from the optical tomographic image acquiring device 1 A of the first comparative example in that the reference optical system includes an optical component 71 disposed midway the optical fiber 51, and that the measurement optical system includes an optical component 72 disposed midway the optical fiber 52. The optical components 71 and 72 are each, e.g., a polarization controller or an attenuator.

In the second comparative example, it is assumed that reflected lights are generated from the optical components 71 and 72. Those reflected lights may also reach the detector 12 through the optical fibers 51 and 52, the coupler 30, and the circulator 20.

The distance along an optical path between the coupler 30 and the emergent end of the optical fiber 51 is denoted by Lr2. The distance along an optical path between the coupler 30 and the emergent end of the optical fiber 52 is denoted by Ls2. The distance along an optical path between the emergent end of the optical fiber 51 and the reference mirror 91 is denoted by Lr1. The distance along an optical path between the emergent end of the optical fiber 52 and the measurement object 92 is denoted by Ls1. The distance along an optical path between the coupler 30 and the optical component 71 is denoted by dr. The distance along an optical path between the coupler 30 and the optical component 72 is denoted by ds. The effective refractive index of the optical fibers 51 and 52 is denoted by n.

On those assumptions, a difference $\Delta L$ between an optical path length $L_{0ref}$ from the coupler 30 to the detector 12 through a path going to and returned from the reference mirror 91 and an optical path length $L_{0obj}$ from the coupler 30 to the detector 12 through a path going to and returned from the measurement object 92 is expressed by the following formula (5a):

$$\Delta L = |L_{0obj} - L_{0ref}|$$

$$= 2|n(Ls2 - Lr2) + (Ls1 - Lr1)|. \qquad \qquad …(5a)$$

Furthermore, a difference $\Delta d$ between an optical path length $L_{2ref}$ from the coupler 30 to the detector 12 through a path going to and returned from the optical component 71 and an optical path length $L_{2obj}$ from the coupler 30 to the detector 12 through a path going to and returned from the optical component 72 is expressed by the following formula (5b):

$$\Delta d = |L_{2obj} - L_{2ref}|$$

$$= 2|n(ds - dr)|. \qquad \qquad …(5b)$$

In the second comparative example, as expressed by the above formula (3), $\Delta L/2$ and $\Delta d/2$ are both smaller than the measurement range width $z_{max}$. Thus, an artifact attributable to the reflected lights generated from the optical components 71 and 72 is superimposed as noise on an optical tomographic image of the measurement object 92 (see Fig. 3(a)).

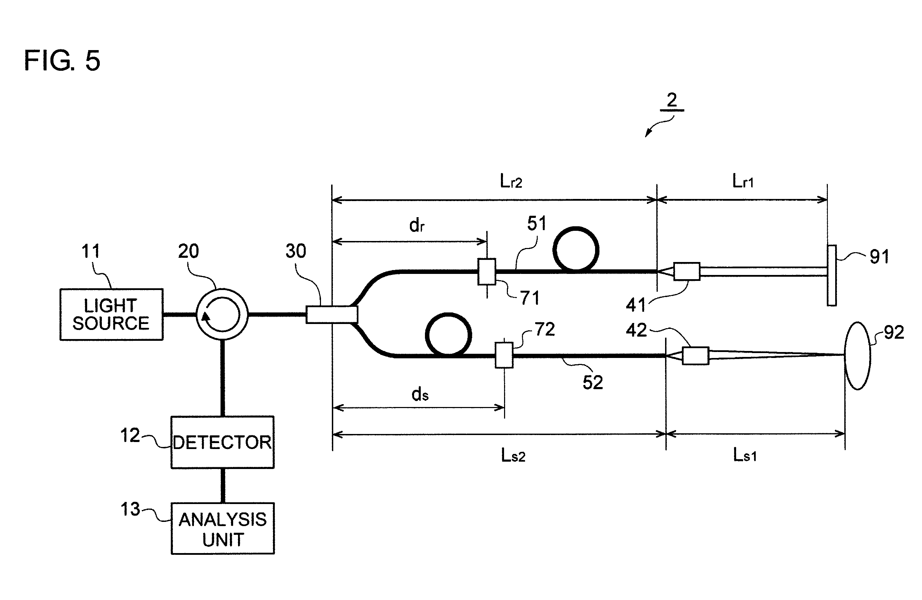

Figure 5 is a conceptual view of an optical tomographic image acquiring device 2 according to a second embodiment. The optical tomographic image acquiring device 2 of the second embodiment is different from the optical tomographic image acquiring device 2A of the second comparative example in that a length (Lr2 - dr) of a portion of the optical fiber 51 in the reference optical system between the optical component 71 and the emergent end of the optical fiber 51 is increased, and that a length ds of a portion of the optical fiber 52 in the measurement optical system between the coupler 30 and the optical component 72 is also increased. When respective changes of the optical path lengths resulting from increases of (Lr2 - dr) and ds are equal to other, $\Delta L$ expressed by the above formula (5a) is not changed and the optical tomographic image of the measurement object 91 is obtained in the second embodiment at the same position as that obtained in the second comparative example.

Furthermore, in the second embodiment, as expressed by the above formula (4), $\Delta L/2$ remains smaller than the measurement range width $z_{max}$, but $\Delta d/2$ is larger than the measurement range width $z_{max}$. In such a case, the artifact attributable to the reflected lights generated from the optical components 71 and 72 is not superimposed on the optical tomographic image of the measurement object 92 (see Fig. 3(b)).

Third comparative example, Third embodiment

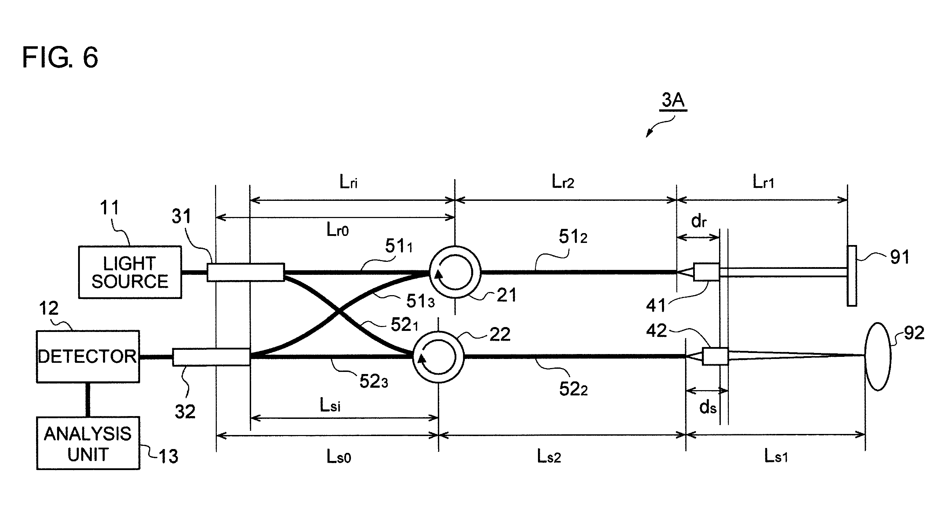

Figure 6 is a conceptual view of an optical tomographic image acquiring device 3A of a third comparative example. The optical tomographic image acquiring device 3A of the third comparative example includes a light source 11, a detector 12, an analysis unit 13, circulators 21 and 22, couplers 31 and 32, a first condensing lens 41, a second condensing lens 42, a first optical fiber $51_1$ to $51_3$, a second optical fiber $52_1$ to $52_3$, and a reference mirror 91. The optical tomographic image acquiring device 3A obtains an optical tomographic image of a measurement object 92 with those components.

The coupler 31 receives light output from the light source 11 and reaching there, and branches the received light into two beams of reference light and measurement light. The coupler 31 outputs the reference light to the optical fiber $51_1$ and the measurement light to the optical fiber $52_1$.

The circulator 21 receives the reference light output from the coupler 31 and reaching there after being guided to propagate through the optical fiber $51_1$, and outputs the reference light to the optical fiber $51_2$. The optical fiber $51_2$ receives at its one end the reference light output from the circulator 21 and outputs the reference light from the other end after guiding the reference light to propagate therethrough. The condensing lens 41 collimates the reference light output from the optical fiber $51_2$ to be incident on the reference mirror 91. Furthermore, the condensing lens 41 receives reflected light generated from the reference mirror 91 upon the incidence of the reference light, and condenses the reflected light to the end surface of the optical fiber $51_2$. The optical fiber $51_2$ outputs the reflected light to the circulator 21 after guiding the reflected light to propagate therethrough. The circulator 21 receives the reflected light output from the optical fiber $51_2$ and outputs the reflected light to the optical fiber $51_3$.

The circulator 22 receives the measurement light output from the coupler 31 and reaching there after being guided to propagate through the optical fiber $52_1$, and outputs the measurement light to the optical fiber $52_2$. The optical fiber $52_2$ receives at its one end the measurement light output from the circulator 22 and outputs the measurement light from the other end after guiding the measurement light to propagate therethrough. The condensing lens 42 condenses the measurement light output from the optical fiber $52_2$ to be applied to the measurement object 92 for irradiation. Furthermore, the condensing lens 42 receives light (object light) reflected from the measurement object 92 upon the irradiation with the measurement light, and condenses the object light to the end surface of the optical fiber $52_2$. The optical fiber $52_2$ outputs the object light to the circulator 22 after guiding the object light to propagate therethrough. The circulator 22 receives the object output from the optical fiber $52_2$ and outputs the object light to the optical fiber $52_3$.

The coupler 32 receives not only the reflected light output from the circulator 21 and reaching there after being guided to propagate through the optical fiber $51_3$, but also the object light output from the circulator 22 and reaching there after being guided to propagate through the optical fiber $52_3$. The coupler 32 outputs interference light, resulting from interference between both the received lights, to the detector 12. The detector 12 receives the interference light output from the coupler 32 and reaching there, and detects the interference light. The analysis unit 13 obtains an optical tomographic image of the measurement object 92 based on the result detected by the detector 12.

In the third comparative example, it is assumed that reflected lights are generated from the condensing lenses 41 and 42. Those reflected lights may also reach the detector 12 through the optical fibers $51_2$ and $52_2$, the circulators 21 and 22, the optical fibers $51_3$ and $52_3$, and the coupler 32.

The distance along an optical path between the coupler 31 and the circulator 21 is denoted by Lri. The distance along an optical path between the coupler 31 and the circulator 22 is denoted by Lsi. The distance along an optical path between the circulator 21 and the emergent end of the optical fiber $51_2$ is denoted by Lr2. The distance along an optical path between the circulator 22 and the emergent end of the optical fiber $52_2$ is denoted by Ls2. The distance along an optical path between the emergent end of the optical fiber $51_2$ and the reference mirror 91 is denoted by Lr1. The distance along an optical path between the emergent end of the optical fiber $52_2$ and the measurement object 92 is denoted by Ls1. The distance along an optical path between the circulator 21 and the coupler 32 is denoted by Lro. The distance along an optical path between the circulator 22 and the coupler 32 is denoted by Lso. The distance along an optical path between the emergent end of the optical fiber $51_2$ and an arbitrary reflecting surface associated with the condensing lens 41 is denoted by dr. The distance along an optical path between the emergent end of the optical fiber $52_2$ and an arbitrary reflecting surface associated with the condensing lens 42 is denoted by ds. The effective refractive index of the optical fibers 51 and 52 is denoted by n.

On those assumptions, a difference $\Delta L$ between an optical path length $L_{0ref}$ from the coupler 31 to the detector 12 through a path going to and returned from the reference mirror 91 and an optical path length $L_{0obj}$ from the coupler 31 to the detector 12 through a path going to and returned from the measurement object 92 is expressed by the following formula (6a):

$$\Delta L = |L_{0obj} - L_{0ref}|$$
$$= |n(Lsi + 2Ls2 + Lso - Lri - 2Lr2 - Lro) + 2(Ls1 - Lr1)|. \quad \dots(6a)$$

Furthermore, a difference $\Delta d$ between an optical path length $L_{1ref}$ from the coupler 31 to the detector 12 through a path going to and returned from the reflecting surface associated with the condensing lens 41 and an optical path length $L_{1obj}$ from the coupler 31 to the detector 12 through a path going to and returned from the reflecting surface associated with the condensing lens 42 is expressed by the following formula (6b):

$$\Delta d = |L_{1obj} - L_{1ref}|$$
$$= |n(Lsi + 2Ls2 + Lso - Lri - 2Lr2 - Lro) + 2(ds - dr)|. \quad \dots(6b)$$

In the third comparative example, as expressed by the above formula (3), $\Delta L/2$ and $\Delta d/2$ are both smaller than the measurement range width $z_{max}$, and an artifact attributable to the reflected lights generated from the condensing lenses 41 and 42 is superimposed as noise on an optical tomographic image of the measurement object 92 (see Fig. 3(a)).

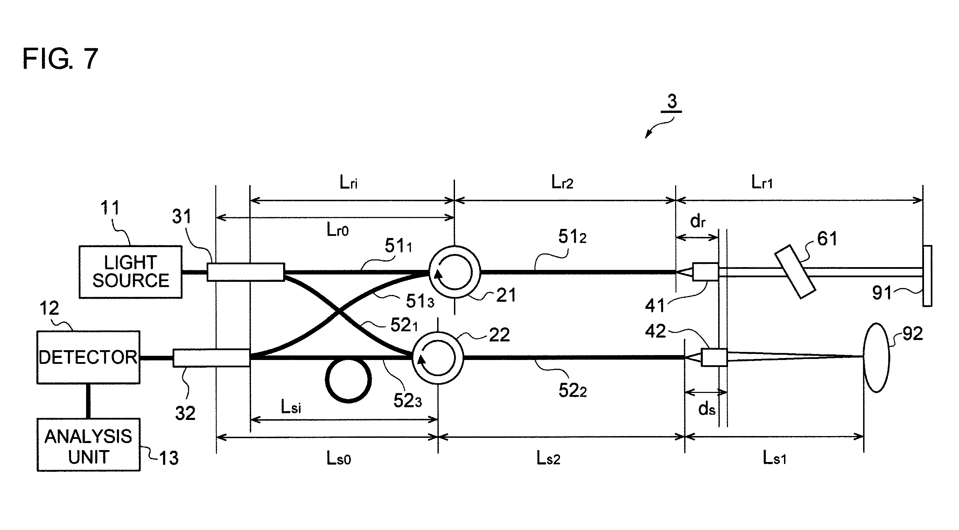

Figure 7 is a conceptual view of an optical tomographic image acquiring device 3 according to a third embodiment. The optical tomographic image acquiring device 3 of the third embodiment is different from the optical tomographic image acquiring device 3A of the third comparative example in that the distance Lr1 along the optical path between the emergent end of the optical fiber $51_2$ and the reference mirror 91 in the reference optical system is increased, and that the length Lso of the optical fiber $52_3$ in the measurement optical system is also increased. When respective changes of the optical path lengths resulting from increases of Lr1 and Lso are equal to other, $\Delta L$ expressed by the above formula (6a) is not changed and the optical tomographic image of the measurement object 91 is obtained in the third embodiment at the same position as that obtained in the third comparative example.

Furthermore, in the third embodiment, as expressed by the above formula (4a), $\Delta L/2$ remains smaller than the measurement range width $z_{max}$, but $\Delta d/2$ is larger than the measurement range width $z_{max}$. In such a case, the artifact attributable to the reflected lights generated from the condensing lenses 41 and 42 is not superimposed on the optical tomographic image of the measurement object 92 (see Fig. 3(b)).

In the third embodiment, because the optical fiber 51 in the reference optical system and the optical fiber 52 in the measurement optical system have different lengths from each other, the influences of dispersions in the optical fibers 51 and 52 are apt to appear in the optical tomographic image. To cope with that problem, a dispersion compensation element 61 is preferably inserted in the optical path between the condensing lens 41 and the reference mirror 91 in the reference optical system. As an alternative, it is also preferable in SD-OCT or SS-OCT to multiply the interference spectrum by a phase component reversed to that of the dispersion.

Fourth comparative example, Fourth embodiment

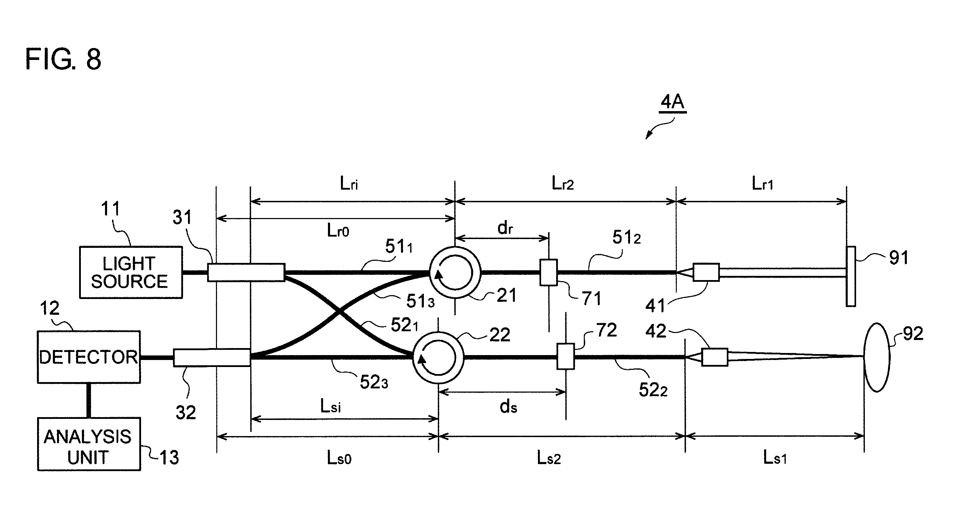

Figure 8 is a conceptual view of an optical tomographic image acquiring device 4A of a fourth comparative example. The optical tomographic image acquiring device 4A of the fourth comparative example is different from the optical tomographic image acquiring device 3A of the third comparative example in that the reference optical system includes an optical component 71 disposed midway the optical fiber $51_2$, and that the measurement optical system includes an optical component 72 disposed midway the optical fiber $52_2$. The optical components 71 and 72 are each, e.g., a polarization controller or an attenuator.

In the fourth comparative example, it is assumed that reflected lights are generated from the optical components 71 and 72. Those reflected lights may also reach the detector 12 through the optical fibers $51_2$ and $52_2$, the circulators 21 and 22, the optical fibers $51_3$ and $52_3$, and the coupler 32.

The distance along an optical path between the coupler 31 and the circulator 21 is denoted by Lri. The distance along an optical path between the coupler 31 and the circulator 22 is denoted by Lsi. The distance along an optical path between the circulator 21 and the emergent end of the optical fiber $51_2$ is denoted by Lr2. The distance along an optical path between the circulator 22 and the emergent end of the optical fiber $52_2$ is denoted by Ls2. The distance along an optical path between the emergent end of the optical fiber $51_2$ and the reference mirror 91 is denoted by Lr1. The distance along an optical path between the emergent end of the optical fiber $52_2$ and the measurement object 92 is denoted by Ls1. The distance along an optical path between the circulator 21 and the coupler 32 is denoted by Lro. The distance along an optical path between the circulator 22 and the coupler 32 is denoted by Lso. The distance along an optical path between the circulator 21 and the optical component 71 is denoted by dr. The distance along an optical path between the circulator 22 and the optical component 72 is denoted by ds. The effective refractive index of the optical fibers 51 and 52 is denoted by n.

On those assumptions, a difference $\Delta L$ between an optical path length $L_{0ref}$ from the coupler 31 to the detector 12 through a path going to and returned from the reference mirror 91 and an optical path length $L_{0obj}$ from the coupler 31 to the detector 12 through a path going to and returned from the measurement object 92 is expressed by the following formula (7a):

$$\Delta L = |L_{0obj} - L_{0ref}|$$
$$= |n(Lsi + 2Ls2 + Lso - Lri - 2Lr2 - Lro) + 2(Ls1 - Lr1)|. \quad \ldots(7a)$$

Furthermore, a difference $\Delta d$ between an optical path length $L_{3ref}$ from the coupler 31 to the detector 12 through a path going to and returned from the optical component 71 and an optical path length $L_{3obj}$ from the coupler 31 to the detector 12 through a path going to and returned from the optical component 72 is expressed by the following formula (7b):

$$\Delta d = |L_{3obj} - L_{3ref}|$$
$$= |n(Lsi + Lso - Lri - Lro) + 2(ds - dr)|. \quad \ldots(7b)$$

In the fourth comparative example, as expressed by the above formula (3), $\Delta L/2$ and $\Delta d/2$ are both smaller than the measurement range width $z_{max}$, and an artifact attributable to the reflected lights generated from the optical components 71 and 72 is superimposed as noise on an optical tomographic image of the measurement object 92 (see Fig. 3 (a)).

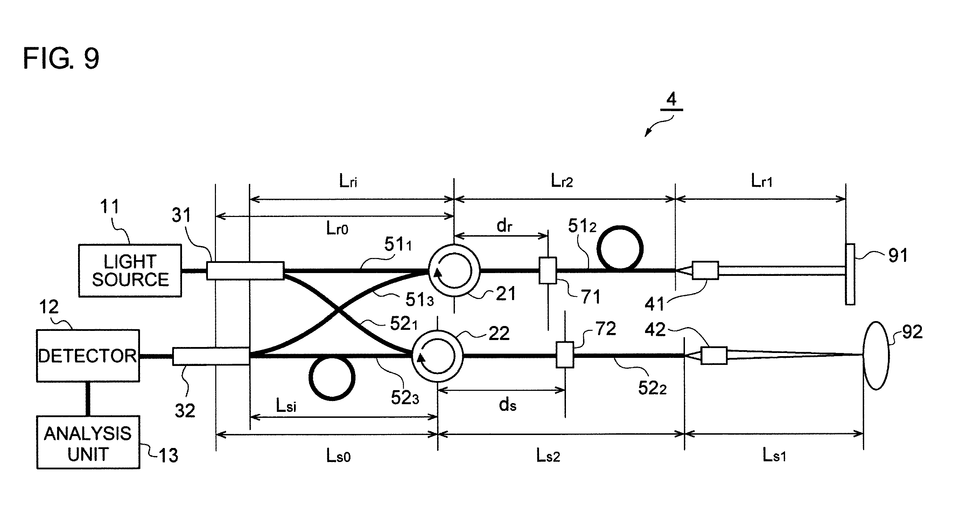

Figure 9 is a conceptual view of an optical tomographic image acquiring device 4 according to a fourth embodiment. The optical tomographic image acquiring device 4 is different from the optical tomographic image acquiring device 4A of the fourth comparative example in that a length (Lr2 - dr) of a portion of the optical fiber $51_2$ in the reference optical system between the optical component 71 and the emergent end of the optical fiber $51_2$ is increased, and that a length Lso of the optical fiber $52_3$ in the measurement optical system is also increased. When respective changes of the optical path lengths resulting from increases of (Lr2 - dr) and ds are equal to other, $\Delta L$ expressed by the above formula (7a) is not changed and the optical tomographic image of the measurement object 91 is obtained in the fourth embodiment at the same position as that obtained in the fourth comparative example.

Furthermore, in the fourth embodiment, as expressed by the above formula (4), $\Delta L/2$ remains smaller than the measurement range width $z_{max}$, but $\Delta d/2$ is larger than the measurement range width $z_{max}$. In such a case, the artifact attributable to the reflected lights generated from the optical components 71 and 72 is not superimposed on the optical tomographic image of the measurement object 92 (see Fig. 3(b)).

Modifications

The present invention is not limited to the above-described embodiments and can be variously modified. In the present invention, it is just required to set the optical path lengths of the reference optical system and the measurement optical system and to set the optical path lengths between the positions where reflected lights causing the artifact are generated (e.g., the condensing lenses or other optical components) and each of the light source and the detector such that the above-mentioned formula (4) is satisfied. Accordingly, there are various ways in adjusting lengths of optical path lengths in which portions of the reference optical system and the measurement optical system.

Industrial Applicability

The optical tomographic image acquiring device is used as an instrument for use in ophthalmology and for observing the lumen of a bored body.

**Claims**

1. An optical tomographic image acquiring device comprising:

   a light source that outputs light;
   a branching member that branches the light output from the light source into two beams of reference light and measurement light;
   a reference optical system including a first optical fiber, a first condensing lens, and a reference mirror, and constituted such that the reference light output from the branching member is guided to propagate through the first optical fiber to be incident on the reference mirror via the first condensing lens, and that reflected light generated from the reference mirror upon the incidence of the reference light is guided to propagate through the first optical fiber via the first condensing lens;
   a measurement optical system including a second optical fiber and a second condensing lens, and constituted such that the measurement light output from the branching member is guided to propagate through the second optical fiber to be applied to the measurement object for irradiation via the second condensing lens, and that reflected light generated from the measurement object upon the irradiation with the measurement light is guided to propagate as object light through the second optical fiber via the second condensing lens;
   a detector that receives interference light resulting from interference between the reflected light output from the reference optical system and the object light output from the measurement optical system, and that detects a spectrum of the interference light by a spectrometer including a plurality of light receiving elements set in array; and
   an analysis unit that obtains an optical tomographic image of the measurement object based on a result detected by the detector,
   wherein, given that $\delta k$ is a maximum value of intervals in wavenumber of lights received by adjacent two of the plural light receiving elements in the spectrometer, an optical path length $L_{0ref}$ from the branching member to the detector through a path going to and returned from the reference mirror and an optical path length $L_{0obj}$ from the branching member to the detector through a path going to and returned from the measurement object satisfy

$$|L_{0obj} - L_{0ref}| < \pi/\delta k$$

   and,
   an optical path length $L_{1ref}$ from the branching member to the detector through a path going to and returned from the first condensing lens and an optical path length $L_{1obj}$ from the branching member to the detector through a path going to and returned from the second condensing lens satisfy:

$$|L_{1obj} - L_{1ref}| > \pi/\delta k.$$

2. An optical tomographic image acquiring device comprising:

a wavelength-variable light source that outputs light;
a branching member that branches the light output from the light source into two beams of reference light and measurement light;
a reference optical system including a first optical fiber, a first condensing lens, and a reference mirror, and constituted such that the reference light output from the branching member is guided to propagate through the first optical fiber to be incident on the reference mirror via the first condensing lens, and that reflected light generated from the reference mirror upon the incidence of the reference light is guided to propagate through the first optical fiber via the first condensing lens;
a measurement optical system including a second optical fiber and a second condensing lens, and constituted such that the measurement light output from the branching member is guided to propagate through the second optical fiber to be applied to the measurement object for irradiation via the second condensing lens, and that reflected light generated from the measurement object upon the irradiation with the measurement light is guided to propagate as object light through the second optical fiber via the second condensing lens;
a detector that receives interference light resulting from interference between the reflected light output from the reference optical system and the object light output from the measurement optical system, and that detects intensity of the interference light at each wavelength of the light output from the wavelength-variable light source; and
an analysis unit that obtains an optical tomographic image of the measurement object based on a result detected by the detector,
wherein, given that $\delta k$ is a maximum value of intervals in wavenumber of light when the intensity of the interference light is detected by the detector, an optical path length $L_{0ref}$ from the branching member to the detector through a path going to and returned from the reference mirror and an optical path length $L_{0obj}$ from the branching member to the detector through a path going to and returned from the measurement object satisfy

$$|L_{0obj} - L_{0ref}| < \pi/\delta k$$

and,
an optical path length $L_{1ref}$ from the branching member to the detector through a path going to and returned from the first condensing lens and an optical path length $L_{1obj}$ from the branching member to the detector through a path going to and returned from the second condensing lens satisfy:

$$|L_{1obj} - L_{1ref}| > \pi/\delta k.$$

3. The optical tomographic image acquiring device according to Claim 1 or 2, wherein the reference optical system includes a first optical component disposed midway the first optical fiber,
the measurement optical system includes a second optical component disposed midway the second optical fiber, and
an optical path length $L_{2ref}$ from the branching member to the detector through a path going to and returned from the first optical component and an optical path length $L_{2obj}$ from the branching member to the detector through a path going to and returned from the second optical component satisfy:

$$|L_{2obj} - L_{2ref}| > \pi/\delta k.$$

4. The optical tomographic image acquiring device according to Claim 1 or 2, wherein the reference optical system includes a first circulator disposed midway the first optical fiber and branching the reflected light generated from the reflection mirror toward the detector, and a first optical component disposed between the first circulator and the first condensing lens,

the measurement optical system includes a second circulator disposed midway the second optical fiber and branching the object light generated from the measurement object toward the detector, and a second optical component disposed between the second circulator and the second condensing lens, and

an optical path length $L_{3ref}$ from the branching member to the detector through a path going to and returned from the first optical component and an optical path length $L_{3obj}$ from the branching member to the detector through a path going to and returned from the second optical component satisfy:

$$|L_{3obj} - L_{3ref}| > \pi/\delta k.$$

FIG. 1

FIG. 2

## FIG. 3

REFLECTION DISTRIBUTION

(a)

0    $\Delta d$    $\Delta L$       $z_{max}$

REFLECTION DISTRIBUTION

(b)

0      $\Delta L$       $z_{max}$

FIG. 4

EP 2 910 925 A1

FIG. 5

FIG. 6

EP 2 910 925 A1

FIG. 7

FIG. 8

4A

LIGHT SOURCE

DETECTOR

ANALYSIS UNIT

FIG. 9

EP 2 910 925 A1

**INTERNATIONAL SEARCH REPORT**

| International application No. |
| --- |
| PCT/JP2013/077299 |

A. CLASSIFICATION OF SUBJECT MATTER
*G01N21/17*(2006.01)i

According to International Patent Classification (IPC) or to both national classification and IPC

B. FIELDS SEARCHED

Minimum documentation searched (classification system followed by classification symbols)
G01N21/17

Documentation searched other than minimum documentation to the extent that such documents are included in the fields searched
Jitsuyo Shinan Koho          1922-1996   Jitsuyo Shinan Toroku Koho   1996-2013
Kokai Jitsuyo Shinan Koho    1971-2013   Toroku Jitsuyo Shinan Koho   1994-2013

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2006-184284 A  (Nippon Telegraph and Telephone Corp.), 13 July 2006 (13.07.2006), entire text; all drawings & WO 2005/031320 A1 | 1-4 |
| A | JP 2010-533301 A  (Volcano Corp.), 21 October 2010 (21.10.2010), entire text; all drawings & WO 2009/009801 A1 | 1-4 |
| A | JP 4-135548 A  (Olympus Optical Co., Ltd.), 11 May 1992 (11.05.1992), entire text; all drawings & US 5305759 A | 1-4 |

| ☒   Further documents are listed in the continuation of Box C. | ☐   See patent family annex. |
| --- | --- |

\*    Special categories of cited documents:
"A"   document defining the general state of the art which is not considered    to be of particular relevance
"E"   earlier application or patent but published on or after the international filing date
"L"   document which may throw doubts on priority claim(s) or which is cited to establish the publication date of another citation or other special reason (as specified)
"O"   document referring to an oral disclosure, use, exhibition or other means
"P"   document published prior to the international filing date but later than the priority date claimed

"T"   later document published after the international filing date or priority date and not in conflict with the application but cited to understand the principle or theory underlying the invention
"X"   document of particular relevance; the claimed invention cannot be considered novel or cannot be considered to involve an inventive step when the document is taken alone
"Y"   document of particular relevance; the claimed invention cannot be considered to involve an inventive step when the document is combined with one or more other such documents, such combination being obvious to a person skilled in the art
"&"   document member of the same patent family

| Date of the actual completion of the international search | Date of mailing of the international search report |
| --- | --- |
| 29 October, 2013 (29.10.13) | 12 November, 2013 (12.11.13) |

| Name and mailing address of the ISA/ Japanese Patent Office | Authorized officer |
| --- | --- |
| Facsimile No. | Telephone No. |

Form PCT/ISA/210 (second sheet) (July 2009)

**INTERNATIONAL SEARCH REPORT**

International application No.

PCT/JP2013/077299

C (Continuation). DOCUMENTS CONSIDERED TO BE RELEVANT

| Category* | Citation of document, with indication, where appropriate, of the relevant passages | Relevant to claim No. |
|---|---|---|
| A | JP 2007-267927 A (Fujifilm Corp.), 18 October 2007 (18.10.2007), entire text; all drawings (Family: none) | 1-4 |

Form PCT/ISA/210 (continuation of second sheet) (July 2009)

**EP 2 910 925 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110299091 A
- JP 2012024551 A